Europäisches Patentamt

European Patent Office

Office européen des brevets

(11) Publication number: **0 497 373 A1**

# EUROPEAN PATENT APPLICATION

(21) Application number: 92101628.3

(22) Date of filing: 31.01.92

(51) Int. Cl.⁵: **G01N 27/06**

(30) Priority: **01.02.91 ZA 910746**

(43) Date of publication of application:
**05.08.92 Bulletin 92/32**

(84) Designated Contracting States:
**AT BE CH DE DK ES FR GB GR IT LI LU MC NL PT SE**

(71) Applicant: **SASOL OIL & FERTILIZERS (PROPRIETARY) LIMITED**
**1, Sturdee Avenue, Rosebank**
**Johannesburg, Transvaal Province(ZA)**

(72) Inventor: **Niemand, Alfonso**
**49 Dee Dee Brown Drive, Wilropark**
**Roodepoort, Transvaal Province(ZA)**

(74) Representative: **Reinhard, Skuhra, Weise**
**Friedrichstrasse 31**
**W-8000 München 40(DE)**

(54) Water detection apparatus.

(57) Portable water detection apparatus 10 comprises a probe 12 which can be lowered into a body of non-aqueous fluid 74. Water sensing means (e.g. two electrodes) is provided in the probe. A flexible connecting member 14 leads from the probe. Indicating means connected to the flexible connecting member remotely from the probe, is also provided. The indicating means is operatively connected to the water sensing means and is adapted to emit a signal when the sensing means are located in water 76, but not when they are located in the non-aqueous fluid 74 (e.g. hydrocarbon liquids such as petrol).

FIG I

THIS INVENTION relates to water detection apparatus. It relates in particular to portable water detection apparatus for detecting the presence of water in a non-aqueous body of fluid, such as liquid hydrocarbons in a storage vessel.

The Applicant is aware that when low viscosity hydrocarbon liquids such as petrol, petrol/alcohol mixtures, diesel, paraffin, aviation fuel and the like, are allowed to stand, eg when stored in storage vessels or tanks, any water present therein will settle out as an aqueous layer at the bottom of the vessel or tank. It is then vital to be able to determine readily whether or not any water has settled out so that the necessary step to remove the water layer, when present, can be taken. Hitherto, the presence of an aqueous layer in the bottom of such tanks has been determined by applying a blob of water indicating paste such as that available under the trade mark KOLOR-KUT M2, from KOLOR-CUT Products, Houston, Texas, USA, to one end of a rod or dipstick, manoeuvering the rod through an access opening in the roof of a hydrocarbon liquid storage vessel until the blob of paste touches the vessel floor, withdrawing the rod, and observing the colour of the paste to determine if there has been a colour change, indicating the presence of a water layer in the bottom of the tank. KOLOR-CUT M2 exhibits a colour change from rust brown to light brown in the presence of water. However, this method is laborious, time consuming, the product often has a limited shelf-life, and the result of the test, ie the colour change, is often dubious.

It is thus an object of this invention to provide a water detection apparatus whereby these drawbacks are at least alleviated.

Thus, according to the invention, there is provided a portable water detection apparatus, which comprises

a portable elongate probe;

water sensing means in the probe;

a flexible connecting member leading from one end of the probe; and

portable indicating means connected to the flexible connecting member remotely from the probe, with the indicating means also being operatively connected to the water sensing means and being adapted to emit a signal when the sensing means are located in water but not when they are located in a non-aqueous liquid.

Thus, in use, the probe can be lowered, eg by means of the flexible connecting member, through an access opening in a vessel or tank holding the non-aqueous body of fluid, which typically is a low viscosity hydrocarbon liquid as hereinbefore described, until the probe reaches the bottom of the tank. Any water which has settled out can then be sensed by the sensing means, with the indicating means indicating the presence of such water.

The water sensing means may comprise a pair of electrodes arranged co-axially in the probe, while the flexible connecting member may be in the form of an electrical cable, with the electrical wires of the cable being connected to the electrodes at one end of the cable. The indicating means may comprise an electronic circuit connected to the electrical wires at the other end of the cable.

The probe may be of elongate form, and may have an outer electrically conductive sleeve which forms the one electrode, with the other electrode extending along the inside of the sleeve. The sleeve may have at least one aperture to permit fluid to enter it.

The cable may be a co-axial cable comprising an electrically conductive metal core; an inner layer of electrically insulative plastics material around the core; an electrically conductive metal mesh around the inner insulative layer; and an outer layer of electrically insulative material around the mesh. The mesh may be connected to the probe sleeve at one end thereof, so that the inner insulative layer and the metal core of the cable protrude into the sleeve towards its other end. The core may be exposed in proximity to the other end of the sleeve so that it constitutes the other electrode. The free end of the core may be anchored in an electrically insulative seal provided at the other end of the sleeve.

The apparatus may include a pocket-sized portable holder connected to the cable at its end remote from the probe, with the holder housing the electronic circuit. The electronic circuit may comprise a first electrical branch connected to the one electrode, as well as to one terminal of a direct current ('DC') electrical battery also housed in the holder, and comprising at least one resistor and a first switch accessible from the outside of the holder, for actuating the circuit; a second electrical branch connected to the other electrode as well as to the other battery terminal, and comprising a resistor and a second switch, also accessible from outside the holder; and a third electrical branch interconnecting the first and second branches and comprising a signal emitter, a further resistor and a suitable transistor as a switching device, with the transistor and resistors being selected such that, on actuating the first and second switches, the signal emitter will be actuated to emit the signal when the electrodes are located in water but not when they are located in a non-aqueous liquid. The signal emitter or alarm may be a light emitting diode ('LED') which lights up when actuated. The signal is hence the lighting up of the LED.

The circuit may include a battery testing branch across the first and second branches and comprising a third switch such that, on actuation of the first, second and third switches, the LED will light up only when there is sufficient battery power.

The invention will now be described by way of example, with reference to the accompanying diagrammatic drawings, in which

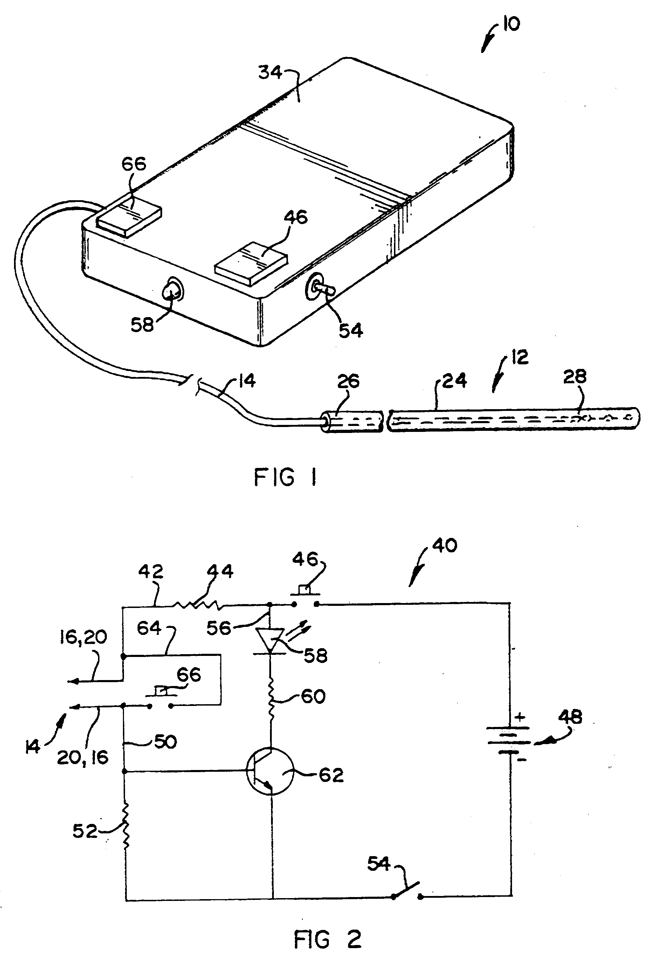

FIGURE 1 shows a three-dimensional view of a portable water detection apparatus in accordance with the invention;

FIGURE 2 shows a schematic circuit diagram for the detector of Figure 1;

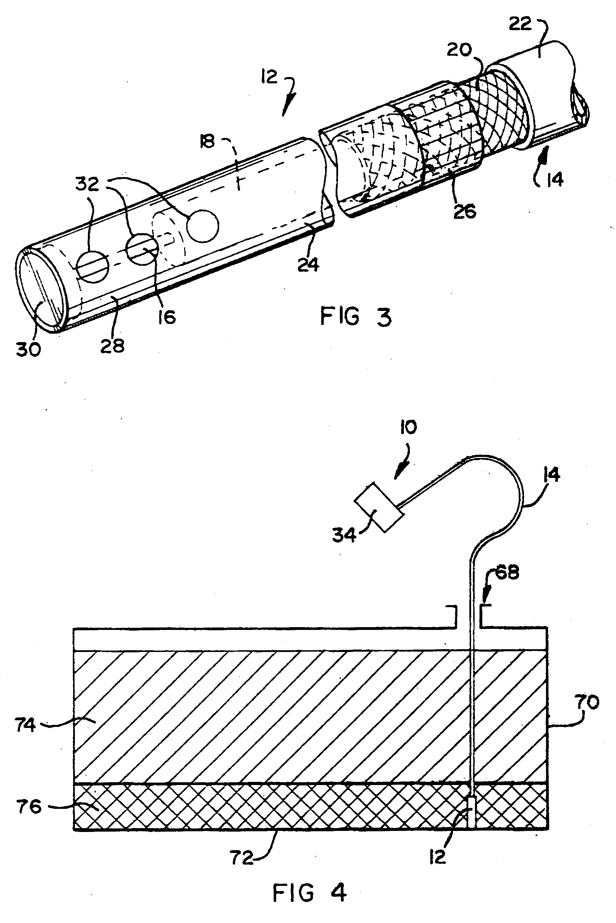

FIGURE 3 shows an enlarged three-dimensional view of a portion of the probe or sensor of the apparatus of Figure 1; and

FIGURE 4 shows, schematically, the apparatus of Figure 1 in use.

In the drawings, reference numeral 10 generally indicates a portable water detection apparatus according to the invention.

The apparatus 10 includes an elongate probe or sensor, generally indicated by reference numeral 12, from which leads a flexible insulated co-axial electric cable 14. The electrical cable 14 comprises an inner metal core 16. Electrically insulative plastics material 18 is located around the core 16, while an outer metal mesh or braid 20 is provided around the inner insulative layer 18. An outer layer 22 of electrically insulative plastics material is provided around the mesh or braid 20.

The probe 12 comprises a stainless steel outer tube 24. To the one end 26 of the tube 24 the metal mesh or braid 20 of the cable 14 is attached, eg by being crimped thereto, so that a portion of the cable protrudes into the tube 24. Thus, the inner insulative layer 18 extends along the tube 24 towards its other end 28, but terminates some distance therefrom. A portion of the cable metal core 16 is exposed in proximity to the tube end 28, and is anchored in a non-electrically conductive seal 30 provided at the tube end 28. A plurality of apertures 32, for permitting fluid to enter the tube 24, are provided in the tube 24 in proximity to its end 28. Typically, the tube 24 can have a length of at least 30 cm, for example between 30 cm and 50 cm, an outer diameter of at least 6 mm, for example between 6 mm and 10 mm, and a wall thickness of about 0,75 mm. The apertures 32 can have a diameter of about 3 mm. A single elongate aperture (not shown) can instead be provided. In addition apertures may be provided on opposite sides of the probe, and may be spaced further apart along the probe.

The apparatus 10 also includes a portable bolder or housing 34 attached to the other end of the electrical cable 14. The holder houses an electrical/electronic circuit, generally indicated by reference numeral 40. The holder 34 is pocket-sized, and, typically has a length of about 8 cm, a width of about 5 cm, and a thickness of about 3 cm. It can also, if desired, be provided with a clip (not shown) for clipping it to a user's belt or the like.

The circuit 40 comprises a first electrical branch 42 attached to either the core 16 or the metal mesh 20 of the cable 14. The branch 42 includes a 1 kΩ resistor 44, and a switch 46. It is connected to the positive terminal of a 9 volt DC electrical battery 48.

The circuit 40 also includes a second electrical branch 50 connected to the other of the cable core 16 or the metal mesh 20. This branch includes a 1 MΩ resistor 52, as well as an on/off switch 54. It is connected to the negative terminal of the battery 48. The branches 42, 50 form a biasing circuit for a switching circuit formed by a further electrical branch 56 which comprises an alarm or indicator in the form of a light emitting diode ('LED') 58, a 1 kΩ resistor 60, and a multi-purposes BC/109/NPN-type transistor 62 as a switching device.

Finally, the circuit 40 includes a battery testing circuit 64 connected to the branches 42, 50 in parallel with the electrodes formed by the tube 24 and core 16. The circuit 64 comprises a switch 66 which, when switched to the on position, effectively shorts out the electrodes. The switches 46, 66 are of a spring-biassed pushbutton type.

In use, the probe 12 is lowered, by means of the cable 14, through an access opening 68 in a liquid hydrocarbon petroleum product storage tank 70, until it touches the base or bottom 72 thereof, at a low point of the base. The probe is thus immersed in the liquid petroleum product 74 in the tank and, assuming that a layer 76 of water has settled out from the hydrocarbon product, will then be located in this water layer 70.

The apparatus 10 is then actuated or switched on by means of the switch 54. Thereafter, the switches 46, 66 are depressed simultaneously to actuate them and to test the battery. If the LED 52 lights up, this will indicate sufficient battery power. The switch 66 can then be released so that only the switch 46 remains depressed, and hence actuated. If there is a water layer 76 present in the tank 70, the LED 58 will again light up. However, in the absence of the water layer 76, the LED will not light up on the switch 46 being depressed.

The operation of the apparatus 10 is thus based, broadly, on two principles. Firstly, it is based thereon that, when water contaminated low viscosity hydrocarbon liquid is stored, the water separates out as a distinct lower layer from the hydrocarbon liquid. Secondly, there is a marked difference in the conductivity of water and liquid hydrocarbon or petroleum products. Thus, when the probe 12 is located in water, the biassing circuit 42, 50 biasses the transistor 62 to the conductive state, thereby actuating the LED 58, while, when the probe is located in hydrocarbon liquid, the transistor 62 is biassed to the non-conductive state. Also, if the battery test switch 66 is actuated, the transistor 62 is biassed to its conductive state.

More specifically, the functioning of the apparatus 10 is based thereon that the electrical resistance of water with which hydrocarbon fuels can be contaminated, such as ground water, is typically about 3500 ohm. The typical electrical resistance of an organic hydrocarbon fuel is in excess of 1000000 ohm. The conductivity, K, of a medium can be defined as follows:

$$K = I \div RA \text{ where}$$

I    is the distance between the electrodes, ie distance between the tube 24 and the core 16, of the probe 12;

R    is the electrical resistance of the medium in question, ie the medium filling the space in the tube around the core 16; and

A    is the contact surface area of the electrodes.

For the dimensions of the probe 12 as stated above, I = 1 mm and A = 30 $mm^2$. With R = 3500 ohm for water, the conductivity, K, of water is thus 9 milli-Siemens/meter. With R being in excess of 1000000 ohm for a typical hydrocarbon petroleum product, the conductivity, K, thereof is less than 0,03 milli-Siemens/meter. The conductivity of water is thus approximately 300 times greater than that of the organic hydrocarbon fuel.

The transistor 62, as soon as an electrical potential of about 0,5 volts or higher is provided between its base and its emitter, conducts an electrical current through its collector and thus through the LED. The minimal current which is necessary for the triggering voltage between the transistor's base and emitter, is provided by the conductivity of the water, via the electrodes of the sensor; however, the conductivity of the hydrocarbon fuel is insufficient to provide sufficient current therefor.

The Applicant believes that the apparatus 10 has substantial advantages over the conventional or known dipstick method of detecting the presence of a water layer in hydrocarbon liquid storage tanks as hereinbefore described. These advantages include ease of use, more accurate and definite determination of the presence of water, can be used in the dark, and has wider applicability, eg can be used to determine water presence in widely differing petroleum products, and even blends, such as petrol and $C_2$-$C_6$ alcohol blends.

The apparatus 10 has the further advantage of being of relatively simple construction and thus being relatively inexpensive to manufacture. Maintenance thereof is also very low, and it is safe to use. The probe 12 can be regenerated easily, if necessary, merely by inserting it in a weak acid solution, such as dilute acetic acid, to dissolve any metal salts which may have deposited on the electrodes as a result of electrolysis. Such deposition of dissolved salts is a general problem when using electrodes, and can affect the reliability of the apparatus over a period of time. The regeneration of the electrodes can easily be effected on the apparatus 10, in view of is portability so that the probe can easily and economically be regenerated as described.

Furthermore, since the apparatus 10 is small, compact and portable, it has multi-purpose application, without requiring any modification or installation. For example, it can be used in any low viscosity water insoluble petroleum products tanks to detect the presence or absence of water.

The apparatus 10 has the further advantage of having the battery testing facility, as hereinbefore described, to ensure accuracy of measurement. A weak battery would give a negative reading indicating the absence of water, ie the LED 58 will then not light up, even if the probe were to be located in a layer of water.

The apparatus 10 is also economical to use in view of its low energy requirements. The apparatus only draws electrical current while a test is being carried out since it is switched off between tests. In addition, the circuit 40 of the apparatus 10 is extremely simple, using only one transistor, and it is not required to connect it to an external alarm system since the LED 62 acts as an alarm.

The apparatus 10 can also be used to indicate the presence of, or the level of water, or any other conductive liquid medium, in other applications such as the level of water in boreholes, water cooling systems, water tanks, other product storage tanks, in quality control laboratories, etc.

The elongate probe 12 permits wide and universal use of the aparatus 10. For example, the probe can be inserted through small openings so that it can be used in limited access applications. The slim-line construction of the probe 12 also permits it to penetrate fluids such as petroleum products easily, and the Applicant has found that a probe 12 of stainless steel, having a length of about 30 cm and a diameter of about 6 cm will satisfactorily penetrate most petroleum fuels.

## Claims

1.    Portable water detection apparatus, characterized in that it comprises

a portable elongate probe;

water sensing means in the probe;

a flexible connecting member leading from one end of the probe; and

portable indicating means connected to the flexible connecting member remotely from the probe, with the indicating means also being operatively connected to the water sensing means and being adapted to emit a signal when the sensing means are located in water but not when they are located in a non-aqueous liquid.

2. Apparatus according to Claim 1, characterized in that the water sensing means comprises a pair of electrodes arranged co-axially in the probe, with the flexible connecting member being in the form of an electrical cable and with the electrical wires of the cable being connected to the electrodes at one end of the cable, and wherein the indicating means comprises an electronic circuit connected to the electrical wires at the other end of the cable.

3. Apparatus according to Claim 2, characterized in that the probe has an outer electrically conductive sleeve which forms the one electrode, with the other electrode extending along the inside of the sleeve, and with the sleeve having at least one aperture to permit fluid to enter it.

4. Apparatus according to Claim 3, characterized in that the cable is a co-axial cable comprising an electrically conductive metal core; an inner layer of electrically insulative material around the core; an electrically conductive metal mesh around the inner insulative layer; and an outer layer of electrically insulative material around the mesh, with the mesh being connected to the probe sleeve at one end thereof, so that the inner insulative layer and the metal core of the cable protrude into the sleeve towards its other end, and with the core being exposed in proximity to the other end of the sleeve so that it constitutes the other electrode, the free end of the core being anchored in an electrically insulative seal provided at the other end of the sleeve.

5. Apparatus according to any one of Claims 2 to 4 inclusive, characterized in that it includes a pocket-sized portable holder connected to the cable at its end remote from the probe, with the holder housing the electronic circuit, and with the electronic circuit comprising a first electrical branch connected to the one electrode, as well as to one terminal of a DC electrical battery also housed in the holder, and comprising at least one resistor and a first switch, accessible from the outside of the holder, for actuating the circuit; a second electrical branch connected to the other electrode as well as to the other battery terminal, and comprising a resistor and a second switch, also accessible from outside the holder; and a third electrical branch interconnecting the first and second branches and comprising a signal emitter, a further resistor and a suitable transistor as a switching device, with the transistor and resistors being selected such that, on actuating the first and second switches, the signal emitter will be actuated to emit the signal when the electrodes are located in water but not when they are located in a non-aqueous liquid.

6. Apparatus according to Claim 5, characterized in that the signal emitter is in the form of a light emitting diode ('LED') which lights up when actuated and wherein the circuit includes a battery testing branch across the first and second branches and comprising a third switch such that, on actuation of the first, second and third switches, the LED will light up only when there is sufficient battery power.

FIG 1

FIG 2

FIG 3

FIG 4

| DOCUMENTS CONSIDERED TO BE RELEVANT | | EP 92101628.3 |

| Category | Citation of document with indication, where appropriate, of relevant passages | Relevant to claim | CLASSIFICATION OF THE APPLICATION (Int. Cl.5) |
|---|---|---|---|
| X | US - A - 4 728 924 (FRANKLIN) * Totality * | 1,2 | G 01 N 27/06 |
| Y | | 3 | |
| A | | 5,6 | |
| A | DE - B - 2 107 088 (ZELLWEGER) * Fig. 1 * | 2 | |
| Y | | 3 | |
| A | DE - A - 1 798 370 (ALCO) * Fig. 2 * | 3 | |
| A | US - A - 3 582 930 (WILEY) * Abstract; fig. 1 * | 5,6 | |
| A | US - A - 4 069 716 (VANASCO) * Abstract; fig. * | 1-6 | TECHNICAL FIELDS SEARCHED (Int. Cl.5) G 01 N G 01 F G 08 B |

The present search report has been drawn up for all claims

| Place of search | Date of completion of the search | Examiner |
|---|---|---|
| VIENNA | 30-04-1992 | NARDAI |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
........................................................................
& : member of the same patent family, corresponding
    document

EPO FORM 1503 03.82 (P0401)